# EUROPEAN PATENT APPLICATION

(11) **EP 4 772 595 A1**
(43) Date of publication of application: **08.07.2026**
(21) Application number: 24859308.9
(22) Date of filing: 31.07.2024
(51) Int. Cl.: C09K 5/10, C07C 211/15

(54) **METHOD FOR SEPARATING PERFLUOROTRIPROPYLAMINE, HEAT TRANSFER FLUID, HEAT TRANSFER DEVICE, AND HEAT TRANSFER METHOD**

(30) Priority: 31.08.2023 JP 2023140902; 19.04.2024 JP 2024068665
(71) Applicant: Daikin Industries, Ltd., Osaka-shi, Osaka 530-0001 (JP)
(72) Inventor: MARI, Daichi, Osaka-Shi, Osaka 530-0001 (JP); SUZUKI, Yuki, Osaka-Shi, Osaka 530-0001 (JP); SHIRAI, Atsushi, Osaka-Shi, Osaka 530-0001 (JP)
(74) Representative: Hoffmann Eitle
(86) International application number: PCT/JP2024/027346
(87) International publication number: WO 2025/047266

(57) **Abstract**

The present disclosure provides a method of separating perfluorotripropylamine from a mixture B, the mixture B containing perfluorotripropylamine and a mixture A containing at least one selected from the group consisting of a hexafluoropropene trimer represented by C₉F₁₈, a perfluoroalkene ether, and a perfluoropolyether, wherein
a coagulation point of the mixture A is a temperature equal to or less than a coagulation point of the perfluorotripropylamine, and
separating the perfluorotripropylamine from the mixture A is carried out at a temperature equal to or less than the coagulation point of the perfluorotripropylamine.

## Description

### TECHNICAL FIELD

The present disclosure relates to a method of separating perfluorotripropylamine, a heat transfer fluid, a heat transfer apparatus, and a heat transfer method.

### BACKGROUND ART

It is known that perfluorotripropylamine is usable as a heat transfer fluid (PTL 1).

### CITATION LIST

### PATENT LITERATURE

PTL 1: Japanese National Patent Publication No. 2016-505882

### SUMMARY OF INVENTION

### TECHNICAL PROBLEM

As a heat transfer fluid, a hexafluoropropene (HFP) trimer, a perfluoroalkene ether, or a perfluoropolyether is usable as a replacement for perfluorotripropylamine.

Inside an apparatus in which perfluorotripropylamine is used as heat transfer fluid, in order to replace the perfluorotripropylamine by another heat transfer fluid (for example, by a hexafluoropropene trimer), it is possible to pass the another heat transfer fluid through the apparatus. This yields a mixture of the perfluorotripropylamine and the another heat transfer fluid. If it is possible to separate the perfluorotripropylamine from this mixture, it is possible to reuse the heat transfer fluid used for replacing.

The present invention aims at providing a separation method capable of efficiently separating perfluorotripropylamine from a mixture containing perfluorotripropylamine and at least one selected from the group consisting of a hexafluoropropene trimer represented by C₉F₁₈, a perfluoroalkene ether, and a perfluoropolyether.

### SOLUTION TO PROBLEM

The present disclosure includes below-described aspects.
[1] A method of separating perfluorotripropylamine from a mixture B, the mixture B containing perfluorotripropylamine and a mixture A containing at least one selected from the group consisting of a hexafluoropropene trimer represented by C₉F₁₈, a perfluoroalkene ether, and a perfluoropolyether, wherein
   a coagulation point of the mixture A is a temperature equal to or less than a coagulation point of the perfluorotripropylamine, and
   separating the perfluorotripropylamine from the mixture B is carried out at a temperature equal to or less than the coagulation point of the perfluorotripropylamine.
[2] The method of separating perfluorotripropylamine according to [1] above, wherein a difference between the coagulation point of the mixture A and the coagulation point of the perfluorotripropylamine is 20°C or more.
[3] The method of separating perfluorotripropylamine according to [1] or [2] above, wherein a content of the perfluorotripropylamine in a mixture C is 1000 ppm by mass or less and the mixture C is a mixture obtained after the perfluorotripropylamine is separated.
[4] The method of separating perfluorotripropylamine according to any one of [1] to [3] above, wherein the hexafluoropropene trimer represented by C₉F₁₈ includes at least one of hexafluoropropene trimers represented by formulae (I) to (III) below. 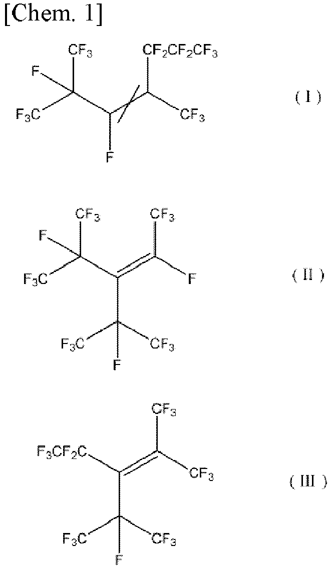
[5] The method of separating perfluorotripropylamine according to any one of [1] to [4] above, wherein the perfluoroalkene ether includes at least one of compounds represented by formulae below:

   CF₃(CF₂)ₓCF=CFCF(OR¹)(CF₂)_{y}CF₃;

   CF₃(CF₂)ₓC(OR¹)=CFCF₂(CF₂)_{y}CF₃;

   CF₃CF=CFCF(OR¹)(CF₂)ₓ(CF₂)_{y}CF₃;

   and

   CF₃(CF₂)ₓCF=C(OR¹)CF₂(CF₂)_{y}CF₃,

   where
   R¹ is independently a methyl group or an ethyl group,
   x and y are each independently 0, 1, 2, or 3, and
   x+y is 1, 2, or 3.
[6] The method of separating perfluorotripropylamine according to any one of [1] to [5] above, wherein the perfluoropolyether includes at least one type of compound represented by a formula below:

   R²O-Rf-R^{2'}

   where
   R and R' are each independently -CₘF₂ₘ₊₁,
   m is an integer of 1 to 8, and
   Rf is a divalent fluoropolyoxyalkylene group containing 2 to 20 repeating units, in which the repeating unit is represented by:
      (i) -CFXO- where X is F or CF₃;
      (ii) -CF₂CFXO- where X is F or CF₃;
      (iii) -CFXCF₂O- where X is F or CF₃;
      (iv) -CF₂CF₂CF₂O-; and/or
      (v) -CF₂CF₂CF₂CF₂O-, or
   Rf is a divalent group represented by:
      (vi) -(CF₂)ₖ-CFZ-O-, where k is an integer of 0 to 3, Z is -ORFT₃, RF is a fluoropolyoxyalkylene group containing a certain number of repeating units selected from the group consisting of -CFXO-, -CF₂CFXO-, -CF₂CF₂CF₂O-, and - CF₂CF₂CF₂CF₂O-, the certain number is from 0 to 20, X is independently F or CF₃, and T₃ is a C₁₋₅ perfluoroalkyl group.
[7] A heat transfer fluid comprising:
   at least one selected from the group consisting of a hexafluoropropene trimer represented by C₉F₁₈, a perfluoroalkene ether, and a perfluoropolyether; and
   perfluorotripropylamine, wherein
   a content of the perfluorotripropylamine is 1000 ppm by mass or less.
[8] The heat transfer fluid according to [7] above, wherein the hexafluoropropene trimer represented by C₉F₁₈ includes at least one of hexafluoropropene trimers represented by formulae (I) to (III) below. 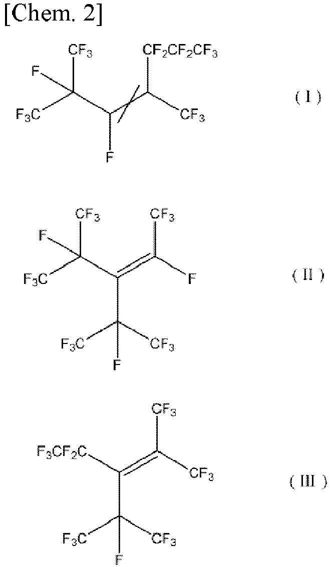
[9] The heat transfer fluid according to [8] above, wherein a compound represented by the formula (I) is contained in 85 mass% or more relative to a total amount of the hexafluoropropene trimer.
[10] The heat transfer fluid according to any one of [7] to [9] above, wherein the perfluoroalkene ether includes at least one of compounds represented by formulae below:

   CF₃(CF₂)ₓCF=CFCF(OR¹)(CF₂)_{y}CF₃;

   CF₃(CF₂)ₓC(OR¹)=CFCF₂(CF₂)_{y}CF₃;

   CF₃CF=CFCF(OR¹)(CF₂)ₓ(CF₂)_{y}CF₃;

   and

   CF₃(CF₂)ₓCF=C(OR¹)CF₂(CF₂)_{y}CF₃,

   where
   R¹ is independently a methyl group or an ethyl group,
   x and y are each independently 0, 1, 2, or 3, and
   x+y is 1, 2, or 3.
[11] The heat transfer fluid according to any one of [7] to [10] above, wherein the perfluoropolyether includes at least one type of compound represented by a formula below:

   R²O-Rf-R^{2'}

   where
   R and R' are each independently -CₘF₂ₘ₊₁,
   m is an integer of 1 to 8,
   Rf is a fluoropolyoxyalkylene chain containing a repeating unit, and
   the repeating unit is represented by:
      (i) -CFXO- where X is F or CF₃;
      (ii) -CF₂CFXO- where X is F or CF₃;
      (iii) -CFXCF₂O- where X is F or CF₃;
      (iv) -CF₂CF₂CF₂O-;
      (v) -CF₂CF₂CF₂CF₂O-; or
      (vi) -(CF₂)ₖ-CFZ-O-, where k is an integer of 0 to 3, Z is -ORFT₃, RF is a fluoropolyoxyalkylene chain containing a certain number of repeating units selected from the group consisting of -CFXO-, -CF₂CFXO-, -CF₂CF₂CF₂O-, and - CF₂CF₂CF₂CF₂O-, the certain number is from 0 to 10, X is independently F or CF₃, and T₃ is a C₁₋₅ perfluoroalkyl group.
[12] The heat transfer fluid according to any one of [7] to [11] above, further comprising a stabilizer.
[13] The heat transfer fluid according to any one of [7] to [12] above usable in a semiconductor production process.
[14] Use of the heat transfer fluid according to any one of [7] to [13] above for transferring heat.
[15] A heat transfer apparatus comprising:
   a device; and
   a mechanism for transferring heat to the device or from the device, wherein the mechanism comprises the heat transfer fluid according to any one of [7] to [13] above.
[16] The heat transfer apparatus according to [15] above, wherein the device is a wafer for use in semiconductor production.
[17] A semiconductor production apparatus comprising the heat transfer apparatus according to [15] or [16] above.
[18] A heat transfer method comprising:
   preparing a device; and
   transferring heat to the device or from the device by using the heat transfer fluid according to any one of [7] to [13] above.
[19] The heat transfer method according to [18] above, wherein the device is a wafer for use in semiconductor production.

### ADVANTAGEOUS EFFECTS OF INVENTION

The present disclosure provides a separation method capable of efficiently separating perfluorotripropylamine from a mixture containing perfluorotripropylamine and at least one selected from the group consisting of a hexafluoropropene trimer represented by C₉F₁₈, a perfluoroalkene ether, and a perfluoropolyether.

### DESCRIPTION OF EMBODIMENTS

Herein, when the expression "each independently" or a similar expression is used for defining a term (a symbol) that can appear multiple times in a chemical structure, this definition applies to every time it appears, independently of one another, whether or not the expression is explicitly described and unless otherwise specified.

In the present specification, a numerical range "(from) A to B" is intended to include both the lower limit numerical value and the upper limit numerical value. In other words, a numerical range "(from) A to B" means not less than A and not more than B.

In the following, a description will be given of a heat transfer fluid according to the present disclosure.

### (Method of Separating Perfluorotripropylamine)

A method of separating perfluorotripropylamine according to the present disclosure is a method of separating perfluorotripropylamine from a mixture B, the mixture B containing perfluorotripropylamine and a mixture A containing at least one selected from the group consisting of a hexafluoropropene trimer represented by C₉F₁₈, a perfluoroalkene ether, and a perfluoropolyether, wherein
a coagulation point of the mixture A is a temperature equal to or less than a coagulation point of the perfluorotripropylamine, and
separating the perfluorotripropylamine from the mixture B is carried out at a temperature equal to or less than the coagulation point of the perfluorotripropylamine. The mixture obtained as a result of treatment performed by the method of separating perfluorotripropylamine according to the present disclosure is also referred to as a mixture C.

The mixture A contains at least one selected from the group consisting of a hexafluoropropene trimer represented by C₉F₁₈, a perfluoroalkene ether, and a perfluoropolyether.

The mixture B is a mixture containing the mixture A and perfluorotripropylamine, and typically it is composed of the mixture A and perfluorotripropylamine. In other words, the mixture A can be regarded as the mixture B from which perfluorotripropylamine is excluded.

The mixture C is a mixture obtained as a result of treatment performed by the method of separating perfluorotripropylamine according to the present disclosure, and it is a mixture obtained by partly or completely removing perfluorotripropylamine from the mixture B.

Typically, the mixture A, the mixture B, and the mixture C may have the same configuration except the content of perfluorotripropylamine. Here, a case where the contents of compounds except perfluorotripropylamine differ is not excluded. For example, the contents (in mass) of individual compounds except perfluorotripropylamine may vary within the range of 10% or less, or 5% or less, or 3% or less, or 1% or less, relative to the entire mixture.

Each of the mixture A, the mixture B, and the mixture C is usable as a heat transfer fluid.

The hexafluoropropene trimer represented by C₉F₁₈ is not limited and may be a compound having any structure represented by C₉F₁₈.

The hexafluoropropene trimer preferably includes at least one of hexafluoropropene trimers represented by formulae (I) to (III) below.

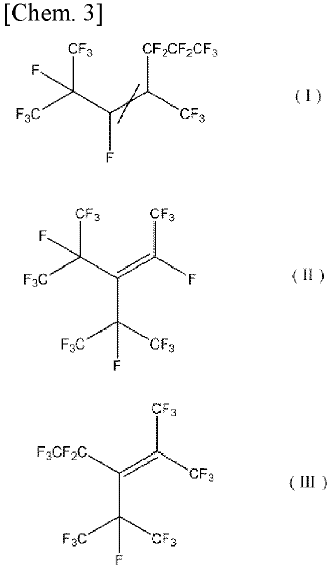

Herein, the compound represented by the formula (I) includes both E- and Z-diastereomers unless otherwise specified.

The hexafluoropropene trimer may be only one of the compounds represented by the formulae (I) to (III), or may be a mixture of two or three of them.

The compound represented by the formula (I) may be contained in 99 mass% or less relative to the total amount of the compounds represented by the formulae (I) to (III), preferably in 90 mass% or less, more preferably in 85 mass% or less, further preferably in less than 85 mass%, and, for example, it may be contained in 80 mass% or less, or in 70 mass% or less, or in 65 mass% or less, or in 60 mass% or less. When the content of the compound represented by the formula (I) is relatively low, the boiling point of the mixture A is relatively high. In other words, the vapor pressure of the mixture A is relatively low.

The compound represented by the formula (I) may be preferably contained in 1 mass% or more relative to the total amount of the compounds represented by the formulae (I) to (III), preferably in 10 mass% or more, more preferably in 30 mass% or more, further preferably in 40 mass% or more, further more preferably in 45 mass% or more, particularly preferably in 50 mass% or more, and, for example, it may be contained in 60 mass% or more, or in 65 mass% or more, or in 75 mass% or more, or in 85 mass% or more. When the content of the compound represented by the formula (I) is relatively high, the viscosity is relatively low.

The compound represented by the formula (I) may be contained in, for example, 1 mass% to 99 mass% relative to the total amount of the compounds represented by the formulae (I) to (III), or in 10 mass% to 90 mass%, or in 30 mass% to 90 mass%, or in 10 mass% to 85 mass%, or in 35 mass% to 85 mass%, or in not less than 10 mass% and less than 85 mass%, or in not less than 20 mass% and less than 85 mass%, or in not less than 30 mass% and less than 85 mass%, or in not less than 40 mass% and less than 85 mass%, or in not less than 50 mass% and less than 85 mass%, or in 40 mass% to 80 mass%, or in 55 mass% to 80 mass%, or in 60 mass% to 75 mass%, or in 65 mass% to 70 mass%, or in 35 mass% to 60 mass%, or in 50 mass% to 60 mass%, preferably in 30 mass% to 90 mass%, preferably in not less than 40 mass% and less than 85 mass%, more preferably in 40 mass% to 80 mass%, further preferably in 45 mass% to 70 mass%, further more preferably in 50 mass% to 60 mass%.

The mass ratio between the compound represented by the formula (II) and the compound represented by the formula (III) is not limited, and, for example, it may be from 1:9 to 9: 1, or from 2:8 to 8:2, or from 3:7 to 7:3, or from 4:6 to 6:4, or from 4.5:5.5 to 5.5:4.5.

The perfluoroalkene ether preferably includes at least one of compounds represented by formulae below:

CF₃(CF₂)ₓCF=CFCF(OR¹)(CF₂)_{y}CF₃;

CF₃(CF₂)ₓC(OR¹)=CFCF₂(CF₂)_{y}CF₃;

CF₃CF=CFCF(OR¹)(CF₂)ₓ(CF₂)_{y}CF₃;

and

CF₃(CF₂)ₓCF=C(OR¹)CF₂(CF₂)_{y}CF₃

[where
R¹ is independently a methyl group or an ethyl group,
x and y are independently 0, 1, 2, or 3, and
x+y is 1, 2, or 3].

The perfluoroalkene ether may be
5-methoxyperfluoro-3-heptene,
3-methoxyperfluoro-3-heptene,
4-methoxyperfluoro-2-heptene,
3-methoxyperfluoro-2-heptene,
4-methoxyperfluoro-2-pentene,
2-methoxyperfluoro-2-pentene,
3-methoxyperfluoro-2-pentene,
2-methoxyperfluoro-3-pentene,
cis- or trans-2-methoxyperfluoro-2-octene, and/or
2-methoxyperfluoro-3-octene, for example.

The perfluoroalkene ether is preferably methylperfluoroheptene ether. The methylperfluoroheptene ether may include a mixture of two or more structural and/or stereoisomers. For example, the methylperfluoroheptene ether may include a mixture containing:
about 48 to about 52 weight percent 5-methoxyperfluoro-3-heptene,
about 18 to about 22 weight percent 3-methoxyperfluoro-3-heptene,
about 18 to about 22 weight percent 4-methoxyperfluoro-2-heptene, and
about 6 to about 10 weight percent 4-methoxyperfluoro-3-heptene.

Specifically, the perfluoroalkene ether includes methyl-perfluoroheptene ether (MPHE) (C₇F₁₃OCH₃). Specific examples include a product under the trade name "Opteon SF10" (manufactured by Chemours) and the like.

The perfluoropolyether includes at least one type of compound represented by a formula below:

R²O-Rf-R^{2'}

where
R and R' are each independently -CₘF₂ₘ₊₁,
m is an integer of 1 to 8, and
Rf is a divalent fluoropolyoxyalkylene group containing 2 to 20 repeating units, in which the repeating unit is represented by:
   (i) -CFXO- where X is F or CF₃;
   (ii) -CF₂CFXO- where X is F or CF₃;
   (iii) -CFXCF₂O- where X is F or CF₃;
   (iv) -CF₂CF₂CF₂O-; and/or
   (v) -CF₂CF₂CF₂CF₂O-, or
Rf is a divalent group represented by:
   (vi) -(CF₂)ₖ-CFZ-O-, where k is an integer of 0 to 3, Z is -ORFT₃, RF is a fluoropolyoxyalkylene group containing a certain number of repeating units selected from the group consisting of -CFXO-, -CF₂CFXO-, -CF₂CF₂CF₂O-, and - CF₂CF₂CF₂CF₂O-, the certain number is from 0 to 20, X is independently F or CF₃, and T₃ is a C₁₋₅ perfluoroalkyl group.

m is an integer of 1 to 8, preferably an integer of 1 to 5 or an integer of 1 to 3.

Preferably, Rf may be groups of (1) to (3) below:

(1) -(CF₂O)ₐ-(CF₂CF₂O)_{b}-(CF₂-(CF₂)_{z'}-CF₂O)_{c}

[where
a, b and c are independently an integer of 100 or less, preferably an integer of 50 or less,
z' is 1 or 2,
a≥0, b≥0, c≥0,
a+b>0, and
preferably a>0, b>0, and b/a is within the range of 0.1 to 10];

(2) -(C₃F₆O)_{c'}-(C₂F₄O)_{b}-(CFXO)ₜ-

-[where
X is independently -F or -CF₃,
b, c', and t are independently an integer of 100 or less,
c'>0, b≥0, t≥0, and
preferably b>0, t>0, c'/b is within the range of 0.2 to 5.0, and (c'+b)/t is within the range of 5 to 50]; and

(3) -(C₃F₆O)_{c'}-(CFXO)ₜ-

-[where
X is independently -F or -CF₃,
c' and t are independently an integer of 100 or less,
c'>0, t≥0, and
preferably t>0 and c'/t is within the range of 5 to 50].

The perfluoropolyether is not limited, and, for example, it may be a product under the trade name GALDEN (registered trademark) HT110 and/or GALDEN (registered trademark) HT135 available from Solvay Solexis S.p.A..

The kinematic viscosity of the mixture A at -75°C may be preferably 100 cSt or less, more preferably 80 cSt or less, further preferably 60 cSt or less. When the mixture A has a kinematic viscosity within the above-mentioned range, namely a relatively low kinematic viscosity, ease of filtration is enhanced. As a result, in a case where micro-filtration is carried out through a filter having fine pore sizes, process time can be reduced and energy loss can be reduced. In addition, filtration with even higher accuracy can be made possible.

The kinematic viscosity and the density of the composition for heat transfer fluid or the heat transfer fluid according to the present disclosure are values measured with a kinematic viscometer, SVM3001, manufactured by Anton Paar.

The boiling point of the mixture A may be preferably 90°C or more, more preferably 95°C or more, further preferably 100°C or more. When the boiling point of the mixture A is high, loss due to evaporation can be reduced.

The boiling point of the mixture A is measured with a differential scanning calorimeter (DSC), as a temperature at which an endothermic peak is observed while the temperature is being raised from 25°C at 5°C/minute.

The coagulation point of the mixture A may be preferably -70°C or less, more preferably -75°C or less, further preferably -80°C or less, further more preferably -85°C or less. The lower the coagulation point of the mixture A is, the greater the difference from the coagulation point of perfluorotripropylamine is and thereby the easier the separation is.

The coagulation point of the mixture A can be determined with the use of a differential scanning calorimeter (DSC).

The coagulation point of perfluorotripropylamine is about -65°C.

The difference between the coagulation point of the mixture A and the coagulation point of perfluorotripropylamine may be preferably 5°C or more, more preferably 10°C or more, further preferably 15°C or more, further more preferably 20°C or more, and, for example, it may be 30°C or more, or 35°C or more, or 40°C or more. When the difference between the coagulation point of the mixture A and the coagulation point of perfluorotripropylamine falls within the above-mentioned range, separation of perfluorotripropylamine is relatively easy to carry out.

The upper limit to the difference between the coagulation point of the mixture A and the coagulation point of perfluorotripropylamine is not limited, and, for example, it may be 100°C or less, or 80°C or less, or 60°C or less, or 50°C or less, or 40°C or less, or 30°C or less.

Separation of perfluorotripropylamine from the mixture B is carried out at a temperature equal to or less than the coagulation point of perfluorotripropylamine.

The temperature at which separation of perfluorotripropylamine from the mixture B is carried out is preferably at least 5°C less than the coagulation point of perfluorotripropylamine, more preferably at least 10°C less, further preferably at least 15°C less, and, for example, it is at least 20°C less, or at least 30°C less.

Preferably, separation of perfluorotripropylamine from the mixture B is carried out at a temperature not more than the coagulation point of perfluorotripropylamine and not less than the coagulation point of the mixture A.

The temperature at which separation of perfluorotripropylamine from the mixture B is carried out is preferably at least 1°C more than the coagulation point of the mixture A, more preferably at least 5°C more, and, for example, it is at least 10°C more, or at least 15°C more.

Preferably, separation of perfluorotripropylamine from the mixture B is carried at a temperature closer to the coagulation point of the mixture A than to the coagulation point of perfluorotripropylamine.

Separation of perfluorotripropylamine from the mixture B is not limited and it may be carried out by extraction, concentration, lyophilization, precipitation, adsorption, distillation, fractionation, or chromatography. Preferably, separation of perfluorotripropylamine from the mixture A may be carried out by lyophilization, recrystallization, adsorption, distillation, fractionation, or chromatography, particularly preferably by adsorption.

Only one of the above-listed methods may be adopted, or a combination of two or more of them may be adopted. For example, a certain method may be adopted to carry out preliminary separation, followed by main separation.

When separation of perfluorotripropylamine from the mixture B is carried out by adsorption, the adsorptive material may be a molecular sieve, activated carbon, silica gel, zeolite, a coordination polymer, a porous polymer, a metal organic framework, porous silica, and/or the like, and a molecular sieve is preferable. Specific examples of the molecular sieve include Molecular Sieve 13X.

The content of perfluorotripropylamine in the mixture C may be preferably 1000 ppm by mass or less, more preferably 500 ppm by mass or less, further preferably 200 ppm by mass or less, further more preferably 100 ppm by mass or less, particularly preferably 50 ppm by mass or less, or 10 ppm by mass or less, or 5 ppm by mass or less, or 1 ppm by mass or less. The mixture C may be substantially free of perfluorotripropylamine. The expression "may be substantially free of perfluorotripropylamine" means that the content of perfluorotripropylamine is equal to or less than the identification limit.

The content of perfluorotripropylamine in the mixture can be analyzed by gas chromatography.

The mixture C preferably contains a hexafluoropropene trimer represented by C₉F₁₈.

The mixture C is preferably a heat transfer fluid. That is, the present disclosure provides a heat transfer fluid comprising perfluorotripropylamine and at least one selected from the group consisting of a hexafluoropropene trimer represented by C₉F₁₈, a perfluoroalkene ether, and a perfluoropolyether, wherein the content of perfluorotripropylamine is 1000 ppm by mass or less.

The heat transfer fluid according to the present disclosure may contain a hexafluoropropene dimer and/or a hexafluoropropene tetramer.

The hexafluoropropene dimer includes (E)-1,1,1,2,3,4,5,5,5-nonafluoro-4-(trifluoromethyl)-2-pentene, (Z)-1,1,1,2,3,4,5,5,5-nonafluoro-4-(trifluoromethyl)-2-pentene, or 1,1,3,4,4,5,5-nonafluoro-2-(trifluoromethyl)-2-pentene.

The hexafluoropropene tetramer includes 1,1,1,2,5,6,6,6-octafluoro-2,3,5-tris(trifluoromethyl)-4-(perfluoropropyl-2-yl)-3-hexene.

The heat transfer fluid according to the present disclosure contains CₘF₂ₘ and/or CₙF₍₂ₙ₋₂₎ [where m is an integer of 4 to 12 except 9, and n is an integer of 4 to 12], in addition to the hexafluoropropene trimer.

m is an integer of 4 or more, preferably an integer of 5 or more, more preferably an integer of 6 or more. Moreover, n is an integer of 12 or less, preferably an integer of 11 or less, more preferably an integer of 10 or less. It should be noted that m does not include 9.

n is an integer of 4 or more, preferably an integer of 5 or more, more preferably an integer of 6 or more. Moreover, n is an integer of 12 or less, preferably an integer of 11 or less, more preferably an integer of 10 or less. Particularly preferably, n is 9.

CₘF₂ₘ may be either a chain compound or a cyclic compound that may have a substituted structure. The chain compound may be a so-called alkene, and it may be either linear or branched.

CₙF₍₂ₙ₋₂₎ may be either a chain compound or a cyclic compound that may have a substituted structure. The chain compound may be a so-called diene or alkyne, and it may be either linear or branched.

When containing both CₘF₂ₘ and/or CₙF₍₂ₙ₋₂₎ and the hexafluoropropene trimer, the heat transfer fluid according to the present disclosure has enhanced functions as a heat transfer fluid. Moreover, when the heat transfer fluid according to the present disclosure contains CₘF₂ₘ and/or CₙF₍₂ₙ₋₂₎, stability of the hexafluoropropene trimer is enhanced.

The content of CₘF₂ₘ and/or CₙF₍₂ₙ₋₂₎ in the heat transfer fluid according to the present disclosure may be preferably 10 mass% or less, more preferably 5 mass% or less, further preferably 1 mass% or less. Moreover, the content of CₘF₂ₘ and/or CₙF₍₂ₙ₋₂₎ in the heat transfer fluid according to the present disclosure may be preferably 0.0001 mass% or more, more preferably 0.001 mass% or more. It should be noted that CₘF₂ₘ and/or CₙF₍₂ₙ₋₂₎ is not an essential component of the heat transfer fluid according to in the present disclosure, and may or may not be contained.

The heat transfer fluid according to the present disclosure may further contain other components.

Other components contained in the heat transfer fluid according to the present disclosure may be any components as long as they do not interfere with the effect and the purpose of the present disclosure. Examples of these other components include water, a stabilizer, and the like.

The stabilizer exhibits stabilizing effects and thereby it functions as a so-called acid scavenger or antioxidant. Examples of major stabilizing effects include effects to capture radicals produced in the system to prevent degradation of the hexafluoropropene trimer, acid scavenging effects to capture acids produced in the system to prevent further degradation of the hexafluoropropene trimer due to acid, and the like.

The stabilizer may be selected from a wide range of known stabilizers. Among these, preferably, for effectively reducing metal corrosion caused by the composition, one or more types of stabilizers selected from the group consisting of unsaturated alcohol-based stabilizers, nitro-based stabilizers, amine-based stabilizers, phenol-based stabilizers, and epoxy-based stabilizers are used.

The unsaturated alcohol-based stabilizer may be selected from a wide range of known ones. For example, one or more selected from the group consisting of 3-buten-2-ol, 2-buten-1-ol, 4-propen-1-ol, 1-propen-3-ol, 2-methyl-3-buten-2-ol, 3-methyl-3-buten-2-ol, 3-methyl-2-buten-1-ol, 2-hexen-1-ol, 2,4-hexadien-1-ol, and oleyl alcohol can be used.

The nitro-based stabilizer may be selected from a wide range of known ones. As aliphatic nitro compounds, nitromethane, nitroethane, 1-nitropropane, 2-nitropropane, and the like may be mentioned, for example. As aromatic nitro compounds, one or more selected from the group consisting of nitrobenzene, o-, m-, and p-dinitrobenzenes, o-, m-, and p-nitrotoluenes, dimethylnitrobenzene, m-nitroacetophenone, o-, m-, and p-nitrophenols, o-nitroanisole, m-nitroanisole, and p-nitroanisole can be used, for example.

The amine-based stabilizer may be selected from a wide range of known ones. For example, one or more selected from the group consisting of pentylamine, hexylamine, diisopropylamine, diisobutylamine, di-n-propylamine, diallylamine, triethylamine, N-methylaniline, pyridine, morpholine, N-methylmorpholine, triallylamine, allylamine, α-methylbenzylamine, dimethylamine, trimethylamine, ethylamine, diethylamine, triethylamine, propylamine, isopropylamine, dipropylamine, tripropylamine, butylamine, isobutylamine, dibutylamine, tributylamine, dipentylamine, tripentylamine, 2-ethylhexylamine, aniline, N,N-dimethylaniline, N,N-diethylaniline, ethylenediamine, propylenediamine, diethylenetriamine, tetraethylenepentamine, benzylamine, dibenzylamine, diphenylamine, and diethylhydroxylamine can be used.

The phenol-based stabilizer may be selected from a wide range of known ones. For example, one or more selected from the group consisting of 2,6-di-tert-butyl-4-methylphenol, 3-cresol, phenol, 1,2-benzenediol, 2-isopropyl-5-methylphenol, and 2-methoxyphenol can be used.

The epoxy-based stabilizer may be selected from a wide range of known ones. For example, one or more selected from the group consisting of butylene oxide, 1,2-propylene oxide, 1,2-butylene oxide, butyl glycidyl ether, diethylene glycol diglycidyl ether, and 1,2-epoxy-3-phenoxypropane can be used.

Use of a combination of stabilizers with different stabilizing effects can more effectively prevent degradation of the hexafluoropropene trimer that can occur from various causes, and therefore, it is preferable to use one or more selected from the group consisting of the epoxy-based stabilizers, the unsaturated alcohol-based stabilizers, the nitro-based stabilizers, and the phenol-based stabilizers mentioned above.

From the viewpoint of effectively reducing acid release from the hexafluoropropene trimer to reduce metal corrosion caused by the liquid composition, the content of the stabilizer in the entire heat transfer fluid is preferably 0.0001 mass% or more, more preferably 0.01 mass% or more. On the other hand, in consideration of avoiding undesired changes of the physical properties of the heat transfer fluid due to excessive addition of stabilizers, the content of the stabilizer in the entire heat transfer fluid is preferably 10 mass% or less, more preferably 5 mass% or less.

### (Use of Heat Transfer Fluid)

The heat transfer fluid according to the present disclosure is used for drawing heat away from various heat transfer targets or supplying heat to heat transfer targets. Examples of the heat transfer target according to the present disclosure include articles, apparatuses, and atmospheres that are to be cooled to, heated to, or maintained at an intended temperature. Examples of the heat transfer target include electric components, machine components, and optical components, as well as those after processing and those after assembly. Specific, non-limiting examples of the heat transfer target according to the present disclosure include wafers used for producing semiconductor devices, microprocessors, power control semiconductors, electric branch switches, power transformers, circuit boards, multi-chip modules, mounted and non-mounted semiconductor devices, chemical reactors, nuclear reactors, fuel cells, lasers, missile components, and the like.

The heat transfer fluid according to the present disclosure is accompanied by a relatively small pressure loss at the time of circulation during use, and therefore suitable for applications where a large amount of heat needs to be transferred. In a preferred aspect, the heat transfer fluid according to the present disclosure is used in a semiconductor production process. In a semiconductor production process, the heat transfer target is a wafer used for producing a semiconductor device.

### (Heat Transfer Apparatus)

The present disclosure further provides a heat transfer apparatus comprising a device and a mechanism for transferring heat to the device or from the device, wherein the mechanism comprises the above-mentioned heat transfer fluid.

Examples of the device include wafers for use in semiconductor production, semiconductor devices, computers, server computers, servers including blade servers; disk array/storage systems; storage area networks; network-attached storages; storage communications systems; workstations; routers; telecommunications infrastructures/switches; wired, optical, and wireless communications apparatuses; cell processing apparatuses; printers; power source apparatuses; displays; optical apparatuses; measurement systems including hand-held systems; military electronic devices; and the like, and preferable among them is a wafer for use in semiconductor production.

Semiconductor devices are heat-generating devices to be mounted on the device, such as CPUs, GPUs, SSDs, and the like, for example, and a semiconductor device is made of a single element such as silicon and germanium, a compound semiconductor such as gallium arsenide (GaAs), gallium phosphide (GaP), indium phosphide (InP), gallium nitride (GaN), and silicon carbide (SiC), and/or the like, for example.

When the device is a server computer, one logic board or a plurality of logic boards are accommodated in the internal space. The logic board comprises many heat-generating electronic components including at least one processor such as CPU and/or GPU. **In** addition to them, other heat-generating components for computers such as those listed below, for example, can be used: chipsets; memories, graphics chips, network chips, RAMs, power source apparatuses, daughter cards; and storage drives such as solid state drives and mechanical hard drives.

The heat transfer apparatus is a heat transfer apparatus that transfers heat to and from a heat transfer target by using the above-mentioned heat transfer fluid, and the supplying and receiving heat (heat transfer) is performed through thermal contact with the heat transfer target. For example, drawing heat away from the heat transfer target is cooling, and supplying heat is heating. Different mechanisms may be used for cooling and heating, respectively, or a singe heat transfer apparatus may be used for both cooling and heating.

The heat transfer apparatus is not limited and, for example, it may be a pump, a valve, a fluid containment system, a pressure control system, a cooler, a heat exchanger, a heat source, a heat sink, a refrigeration system, an active temperature control system, a passive temperature control system, and/or the like.

More specific examples of the heat transfer apparatus include a temperature-controlled wafer chuck installed inside a plasma enhanced chemical vapor deposition (PECVD) tool, a temperature-controlled test head for die performance testing, a temperature-controlled workspace inside a semiconductor process device, a reservoir for thermal shock test bath liquid, a thermostatic chamber, and the like, and preferable among them is a temperature-controlled workspace inside a semiconductor process device.

The heat transfer target to be brought into thermal contact with the heat transfer apparatus is as described above.

The present disclosure also provides a semiconductor production apparatus comprising the heat transfer apparatus according to the present disclosure.

### (Heat Transfer Method)

The present disclosure discloses a heat transfer method comprising a step of preparing a device, and a step of transferring heat to the device or from the device with the use of the above-mentioned heat transfer fluid. Here, the heat transfer apparatus is placed in thermal contact with the device so as to make heat transfer possible. When placed in thermal contact with the device, the heat transfer apparatus can draw heat away from the device, or supply heat to the device, or maintain the device at a selected temperature or within a selected temperature range. The direction of heat flow (from the device or to the device) depends on the relative temperature difference between the device and the heat transfer apparatus.

Thus far, the present invention is described, but the present invention is not limited to what is described above and can be implemented in various configurations without departing from the gist of the present invention.

### Examples

In the following, the present disclosure will be described by way of examples, but the scope of the present disclosure is not limited to these examples.

The content of perfluorotripropylamine in a composition was measured by gas chromatography analysis.

The boiling point and the coagulation point of the composition were measured with a differential scanning calorimeter (DSC). Specifically, the boiling point was a temperature at which an endothermic peak was observed while the temperature was being raised from 25°C at 5°C/min. The coagulation point was a temperature at which an endothermic peak was observed while the temperature was being raised at 5°C/min after lowered with liquid nitrogen to -150°C or less (the temperature at which solidification was observed).

### <Example to Synthesize Heat Transfer Fluid a (Mixture A)>

In an autoclave made of SUS, 750 g of DMF and 7.2 g of cesium fluoride were placed and hermetically sealed. After air was evacuated from the autoclave, 2268 g of hexafluoropropylene was added over 4.5 hours while the temperature inside the autoclave was maintained at 70 to 110°C. From the reaction liquid thus obtained, the lower layer was separated and rinsed with ultrapure water, and thereby 2219 g of a heat transfer fluid composition a containing an HFP trimer was obtained (a mixture A). GC-FID and GC-MS analyses were carried out, and by an area normalization method, it was checked that the content of the HFP trimer was 87 mass% in the total amount of the composition regarded as 100 mass%, and in the total amount of the HFP trimer regarded as 100 mass%, the contents of the compounds represented by the formulae (I), (II), and (III) according to the present specification were 78 mass%, 9 mass%, and 13 mass%, respectively.

### <Production Example 1>

990 g of the heat transfer fluid a (the mixture A) and 10 g of FC-3283 (perfluorotripropylamine) manufactured by 3M were mixed to form a raw material composition 1 (a mixture B).

### <Production Example 2>

990 g of Opteon SF10 manufactured by Chemours (methylperfluoroheptene ether) (a mixture A) and 10 g of FC-3283 (perfluorotripropylamine) manufactured by 3M were mixed to form a raw material composition 2 (a mixture B).

### <Production Example 3>

990 g of Galden HT110 (perfluoropolyether, with a boiling point of 110°C and a coagulation point of -100°C) (a mixture A) manufactured by Solvay and 10 g of FC-3283 (perfluorotripropylamine) manufactured by 3M were mixed to form a raw material composition 3 (a mixture B).

### <Production Example 4>

990 g of Galden HT135 (perfluoropolyether, with a boiling point of 110°C and a coagulation point of -100°C) (a mixture A) manufactured by Solvay and 10 g of FC-3283 (perfluorotripropylamine) manufactured by 3M were mixed to form a raw material composition 4 (a mixture B).

### <Example 1>

1000 g of the raw material composition 1 and 50 g of un-used Molecular Sieve 13X (MS13X) were placed in a flask that was dried in advance. While the temperature of the liquid in the flask was being controlled at -75°C, and with occasional stirring, treatment was allowed to proceed for 20 hours. After a lapse of 20 hours, filtration was carried out, and thereby a post-treatment composition of Example 1 (a mixture C) was obtained. Part of the resulting composition of Example 1 was analyzed by gas chromatography, and the content of perfluorotripropylamine in the composition of Example 1 was calculated to be 483 ppm.

### <Example 2>

1000 g of the raw material composition 1 and 100 g of un-used Molecular Sieve 13X were placed in a flask that was dried in advance. While the temperature of the liquid in the flask was being controlled at -75°C, and with occasional stirring, treatment was allowed to proceed for 20 hours. After a lapse of 20 hours, filtration was carried out, and thereby a post-treatment composition of Example 2 (a mixture C) was obtained. Part of the resulting composition of Example 2 was analyzed by gas chromatography, and the content of perfluorotripropylamine in the composition of Example 2 was calculated to be 234 ppm.

### <Example 3>

1000 g of the raw material composition 1 and 400 g of un-used Molecular Sieve 13X were placed in a flask that was dried in advance. While the temperature of the liquid in the flask was being controlled at -75°C, and with occasional stirring, treatment was allowed to proceed for 20 hours. After a lapse of 20 hours, filtration was carried out, and thereby a post-treatment composition of Example 3 (a mixture C) was obtained. Part of the resulting composition of Example 3 was analyzed by gas chromatography, and the content of perfluorotripropylamine in the composition of Example 3 was calculated to be 1 ppm.

### <Example 4>

1000 g of the raw material composition 2 and 400 g of un-used Molecular Sieve 13X were placed in a flask that was dried in advance. While the temperature of the liquid in the flask was being controlled at -75°C, and with occasional stirring, treatment was allowed to proceed for 20 hours. After a lapse of 20 hours, filtration was carried out, and thereby a post-treatment composition of Example 4 (a mixture C) was obtained. Part of the resulting composition of Example 4 was analyzed by gas chromatography, and the content of perfluorotripropylamine in the composition of Example 4 was calculated to be 6 ppm.

### <Example 5>

1000 g of the raw material composition 3 and 400 g of un-used Molecular Sieve 13X were placed in a flask that was dried in advance. While the temperature of the liquid in the flask was being controlled at -75°C, and with occasional stirring, treatment was allowed to proceed for 20 hours. After a lapse of 20 hours, filtration was carried out, and thereby a post-treatment composition of Example 5 (a mixture C) was obtained. Part of the resulting composition of Example 5 was analyzed by gas chromatography, and the content of perfluorotripropylamine in the composition of Example 5 was calculated to be 8 ppm.

### <Example 6>

1000 g of the raw material composition 4 and 400 g of un-used Molecular Sieve 13X were placed in a flask that was dried in advance. While the temperature of the liquid in the flask was being controlled at -75°C, and with occasional stirring, treatment was allowed to proceed for 20 hours. After a lapse of 20 hours, filtration was carried out, and thereby a post-treatment composition of Example 6 (a mixture C) was obtained. Part of the resulting composition of Example 6 was analyzed by gas chromatography, and the content of perfluorotripropylamine in the composition of Example 6 was calculated to be 3 ppm.

### <Comparative Example 1>

1000 g of the raw material composition 1 and 50 g of un-used Molecular Sieve 13X were placed in a flask that was dried in advance. While the temperature of the liquid in the flask was being controlled at 25°C, and with occasional stirring, treatment was allowed to proceed for 20 hours. After a lapse of 20 hours, filtration was carried out, and thereby a post-treatment composition was obtained. Part of the resulting composition was analyzed by gas chromatography, and the content of perfluorotripropylamine in the composition was calculated to be 5723 ppm.

**[Table 1]**

| Item | | | | Comp. Ex. 1 | Ex. 1 | Ex. 2 | Ex. 3 | Ex. 4 | Ex. 5 | Ex. 6 |
|---|---|---|---|---|---|---|---|---|---|---|
| Raw material | Sample name | | - | Raw material composition 1 | Raw material composition 1 | Raw material composition 1 | Raw material composition 1 | Raw material composition 2 | Raw material composition 3 | Raw material composition 4 |
| | Amount used | | g | 1000 | 1000 | 1000 | 1000 | 1000 | 1000 | 1000 |
| | Mixture A | Content | wt% | 99 | 99 | 99 | 99 | 99 | 99 | 99 |
| | | Boiling point | °C | 105 | 105 | 105 | 105 | 110 | 110 | 135 |
| | | Coagulation point | °C | -90 | -90 | -90 | -90 | -90 | -100 | -100 |
| | FC3283 | Content | wt% | 1 | 1 | 1 | 1 | 1 | 1 | 1 |
| | | Boiling point | °C | 128 | 128 | 128 | 128 | 128 | 128 | 128 |
| | | Coagulation point | °C | -65 | -65 | -65 | -65 | -65 | -65 | -65 |
| | Difference in coagulation point | | °C | 25 | 25 | 25 | 25 | 25 | 35 | 35 |
| Separation conditions | Separation method | | - | Adsorption | Adsorption | Adsorption | Adsorption | Adsorption | Adsorption | Adsorption |
| | Separation conditions | Adsorptive agent | - | MS13X | MS13X | MS13X | MS13X | MS13X | MS13X | MS13X |
| | | Amount used | g | 50 | 50 | 100 | 400 | 400 | 400 | 400 |
| | | Temperature | °C | 25 | -75 | -75 | -75 | -75 | -75 | -75 |
| | | Treatment time | hr | 20 | 20 | 20 | 20 | 20 | 20 | 20 |
| Results | Amount of FC3283 in mixture C | | ppm | 5723 | 483 | 234 | 1 | 6 | 8 | 3 |

### INDUSTRIAL APPLICABILITY

The method of separating perfluorotripropylamine according to the present disclosure is suitable for use in purification of heat transfer fluid.

## Claims

1. A method of separating perfluorotripropylamine from a mixture B, the mixture B containing perfluorotripropylamine and a mixture A containing at least one selected from the group consisting of a hexafluoropropene trimer represented by C₉F₁₈, a perfluoroalkene ether, and a perfluoropolyether, wherein
a coagulation point of the mixture A is a temperature equal to or less than a coagulation point of the perfluorotripropylamine, and
separating the perfluorotripropylamine from the mixture B is carried out at a temperature equal to or less than the coagulation point of the perfluorotripropylamine.

2. The method of separating perfluorotripropylamine according to claim 1, wherein a difference between the coagulation point of the mixture A and the coagulation point of the perfluorotripropylamine is 20°C or more.

3. The method of separating perfluorotripropylamine according to claim 1 or 2, wherein a content of the perfluorotripropylamine in a mixture C is 1000 ppm by mass or less and the mixture C is a mixture obtained after the perfluorotripropylamine is separated.

4. The method of separating perfluorotripropylamine according to any one of claims 1 to 3, wherein the hexafluoropropene trimer represented by C₉F₁₈ includes at least one of hexafluoropropene trimers represented by formulae (I) to (III) below.

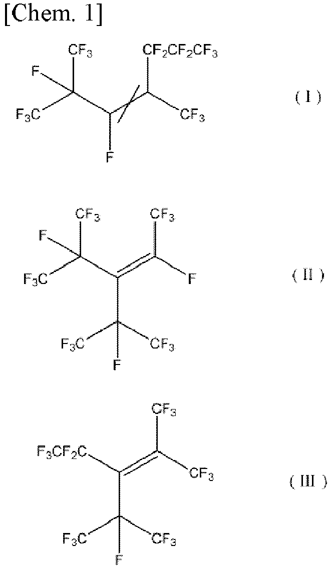

5. The method of separating perfluorotripropylamine according to any one of claims 1 to 4, wherein the perfluoroalkene ether includes at least one of compounds represented by formulae below:
CF₃(CF₂)ₓCF=CFCF(OR¹)(CF₂)_{y}CF₃;
CF₃(CF₂)ₓC(OR¹)=CFCF₂(CF₂)_{y}CF₃;
CF₃CF=CFCF(OR¹)(CF₂)ₓ(CF₂)_{y}CF₃;
and
CF₃(CF₂)ₓCF=C(OR¹)CF₂(CF₂)_{y}CF₃,
where
R¹ is independently a methyl group or an ethyl group,
x and y are each independently 0, 1, 2, or 3, and
x+y is 1, 2, or 3.

6. The method of separating perfluorotripropylamine according to any one of claims 1 to 5, wherein the perfluoropolyether includes at least one type of compound represented by a formula below:
R²O-Rf-R^{2'}
where
R and R' are each independently -CₘF₂ₘ₊₁,
m is an integer of 1 to 8, and
Rf is a divalent fluoropolyoxyalkylene group containing 2 to 20 repeating units, in which the repeating unit is represented by:
(i) -CFXO- where X is F or CF₃;
(ii) -CF₂CFXO- where X is F or CF₃;
(iii) -CFXCF₂O- where X is F or CF₃;
(iv) -CF₂CF₂CF₂O-; and/or
(v) -CF₂CF₂CF₂CF₂O-, or
Rf is a divalent group represented by:
(vi) -(CF₂)ₖ-CFZ-O-, where k is an integer of 0 to 3, Z is -ORFT₃, RF is a fluoropolyoxyalkylene group containing a certain number of repeating units selected from the group consisting of -CFXO-, -CF₂CFXO-, -CF₂CF₂CF₂O-, and - CF₂CF₂CF₂CF₂O-, the certain number is from 0 to 20, X is independently F or CF₃, and T₃ is a C₁₋₅ perfluoroalkyl group.

7. A heat transfer fluid comprising:
at least one selected from the group consisting of a hexafluoropropene trimer represented by C₉F₁₈, a perfluoroalkene ether, and a perfluoropolyether; and
perfluorotripropylamine, wherein
a content of the perfluorotripropylamine is 1000 ppm by mass or less.

8. The heat transfer fluid according to claim 7, wherein the hexafluoropropene trimer represented by C₉F₁₈ includes at least one of hexafluoropropene trimers represented by formulae (I) to (III) below.

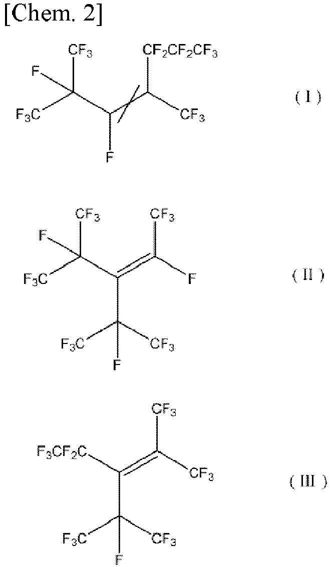

9. The heat transfer fluid according to claim 8, wherein a compound represented by the formula (I) is contained in 85 mass% or more relative to a total amount of the hexafluoropropene trimer.

10. The heat transfer fluid according to any one of claims 7 to 9, wherein the perfluoroalkene ether includes at least one of compounds represented by formulae below:
CF₃(CF₂)ₓCF=CFCF(OR¹)(CF₂)_{y}CF₃;
CF₃(CF₂)ₓC(OR¹)=CFCF₂(CF₂)_{y}CF₃;
CF₃CF=CFCF(OR¹)(CF₂)ₓ(CF₂)_{y}CF₃;
and
CF₃(CF₂)ₓCF=C(OR¹)CF₂(CF₂)_{y}CF₃,
where
R¹ is independently a methyl group or an ethyl group,
x and y are each independently 0, 1, 2, or 3, and
x+y is 1, 2, or 3.

11. The heat transfer fluid according to any one of claims 7 to 10, wherein the perfluoropolyether includes at least one type of compound represented by a formula below:
R²O-Rf-R^{2'}
where
R and R' are each independently -CₘF₂ₘ₊₁,
m is an integer of 1 to 8,
Rf is a fluoropolyoxyalkylene chain containing a repeating unit, and
the repeating unit is represented by:
(i) -CFXO- where X is F or CF₃;
(ii) -CF₂CFXO- where X is F or CF₃;
(iii) -CFXCF₂O- where X is F or CF₃;
(iv) -CF₂CF₂CF₂O-;
(v) -CF₂CF₂CF₂CF₂O-; or
(vi) -(CF₂)ₖ-CFZ-O-, where k is an integer of 0 to 3, Z is -ORFT₃, RF is a fluoropolyoxyalkylene chain containing a certain number of repeating units selected from the group consisting of -CFXO-, -CF₂CFXO-, -CF₂CF₂CF₂O-, and - CF₂CF₂CF₂CF₂O-, the certain number is from 0 to 10, X is independently F or CF₃, and T₃ is a C₁₋₅ perfluoroalkyl group.

12. The heat transfer fluid according to any one of claims 7 to 11, further comprising a stabilizer.

13. The heat transfer fluid according to any one of claims 7 to 12 usable in a semiconductor production process.

14. Use of the heat transfer fluid according to any one of claims 7 to 13 for transferring heat.

15. A heat transfer apparatus comprising:
a device; and
a mechanism for transferring heat to the device or from the device, wherein the mechanism comprises the heat transfer fluid according to any one of claims 7 to 13.

16. The heat transfer apparatus according to claim 15, wherein the device is a wafer for use in semiconductor production.

17. A semiconductor production apparatus comprising the heat transfer apparatus according to claim 15 or 16.

18. A heat transfer method comprising:
preparing a device; and
transferring heat to the device or from the device by using the heat transfer fluid according to any one of claims 7 to 13.

19. The heat transfer method according to claim 18, wherein the device is a wafer for use in semiconductor production.
